# EUROPÄISCHE PATENTANMELDUNG

(11) **EP 2 005 939 A1**
(43) Veröffentlichungstag der Anmeldung: **24.12.2008**
(21) Anmeldenummer: 08009775.1
(22) Anmeldetag: 29.05.2008
(51) Int. Cl.: A61K 8/06, A61K 8/22, A61Q 13/00, A61Q 19/00

(54) **Kosmetische oder dermatologische Zubereitungen mit einem Gehalt an molekularem Sauerstoff und Parfümstoffen**

(30) Priorität: 14.06.2007 DE 102007028024
(71) Anmelder: Beiersdorf AG, 20245 Hamburg (DE)
(72) Erfinder: Zilz, Werner, 25469 Halstenbek (DE); Treu, Jens, 22844 Norderstedt (DE); Blatt, Thomas, 22880 Wedel (DE); Kolbe, Ludger, 21255 Dohren (DE); Fey, Sven, 22397 Hamburg (DE); Rathsack, Jessica, 21614 Buxtehude (DE); Möllgaard, Svenja Lena, 22301 Hamburg (DE)

(57) **Zusammenfassung**

Kosmetische Zubereitungen mit einem Gehalt an molekularem Sauerstoff und einem oder mehreren Parfümstoffen, insbesondere gewählt aus der Gruppe der Verbindungen Methylbenzoat ,3,7-Dimethyl-6-octen-1-ol, 2,6-Dimethyl-7-octen-2-ol, Hydroxycitronellal, omega-Pentadecalacton, Linalool, Coumarin, Bergamotteöl, Lavandinöl, Lavendelöl, Benzylsalicylat, Phenethylalkohol, Linalylacatat, Patchouliöl, alpha-Isomethylionon, Orangenterpene, 4-(4-hydroxy-4-methylpentyl)-3-cyclohexene-1-carboxyaldehyd, Methylcedrylketon, d-Limonene, Limonenöl, alpha-Hexylzimtaldehyd, 1,3,4,6,7,8-Hexahydro-4,8,6,7,8,8-hexamethylcyclopentagamma-2-benzopyran, Terpinylacetat, Ethylenbrassylat, 1,2,3,4,5,6,7,8-octahydro-2,3,8,8-tetramethyl-2-Acetonaphthon, Amylsaliclyat, Terpineol, Cedrol, Vanillin, Methyldihdrojasmonat, Sclareoat, 2-Isobutyl-4-hydroxy-4-methyltetrahydropyran, 3-Methyl-5-phenyl-1-pentanol, Hexylsalicylat, Tetrahydrolinalool, Ethyllinalool, p-t-Butyl-alpha-methyldihydrozimtaldehyd, Benzylacetat, Orangenöl, 7-Acetyl-1,1,3,4,4,6-hexamethyltetralin, 2-tert-Pentyl-cyclohexylacetat, Lavandrinöl, Citronellol, Anisalkohol, Linaool, Heliotropin, d-Limonen, Benzylbenzoat, Amylcinnamylalkohol, Benzylcinnamat, Geraniol, Citral, Eugenol, Isoeugenol Famesol, Methyloctinoat, Linalylacetat, Methylcedrylketon, 4-t-Butylcyclohexylacetet, 3,7-Dimethyl-2,6-octadien-1-ol, Ethylenbrassylat, 3-Methyl-5-phenyl-1-pentanol, Terpinylacetat, Linalylacetat, 2-Isobutyl-4-hydroxy-4-methyltetrahydrofuran, alpha-Amylzimtaldehyd.

## Beschreibung

Die vorliegende Erfindung betrifft eine kosmetische Zubereitung mit einem Gehalt an molekularem Sauerstoff und einem oder mehreren Parfümstoffen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Kosmetika enthalten normalerweise eine Reihe von Parfümstoffen, die dazu dienen sollen, unangenehme Eigengerüche von Zubereitungsbestandteilen zu überdecken und dem Kosmetikum den individuellen, Hersteller-typischen Geruch zu verleihen.

Kosmetika enthalten normalerweise eine Reihe von Parfümstoffen, die dazu dienen sollen, unangenehme Eigengerüche von Zubereitungsbestandteilen zu überdecken und dem Kosmetikum den individuellen, Hersteller-typischen Geruch zu verleihen.

Dies ist jedoch oftmals schwierig, insbesondere bei Anwesenheit bestimmter Füllstoffe, die bedingen, daß eine größere Menge an Parfum zugegeben werden muß.

Da eine Reihe von Parfümbestandteilen aus dermatologischer Sicht nicht vollkommen unproblematisch ist, besteht ein Übelstand, den abzuhelfen eine Aufgabe der vorliegenden Erfindung ist. Insbesondere Limonen, Citral, Linalool, alpha-Isomethylionon. Geraniol und Citronellol können in Einzelfällen bei Anwendern mit besonders empfindlicher Haut zu Hautreizungen, Rötungen und anderen Unverträglichkeitsreaktionen führen.

Es war daher eine weitere Aufgabe der vorliegenden Erfindung die Nachteile des Standes der Technik zu beseitigen und hautfreundlichere parfümierte kosmetische Zubereitungen zu entwickeln.

Überraschend gelöst wird die Aufgabe durch kosmetische Zubereitungen mit einem Gehalt an molekularem Sauerstoff und einem oder mehreren Parfümstoffen, insbesondere gewählt aus der Gruppe der Verbindungen Methylbenzoat ,3.7-Dimethyl-6-octen-1-ol, 2,6-Dimethyl-7-octen-2-ol, Hydroxycitronellal, omega-Pentadecalacton, Linalool, Coumarin, Bergamotteöl, Lavandinöl, Lavendelöl, Benzylsalicylat, Phenethylalkohol, Linalylacatat, Pätchouliöl, alpha-Isomethylionon, Orangenterpene. 4-(4-hydroxy-4-methylpentyl)-3-cyclohexene-1-carboxyaldehyd, Methylcedrylketon, d-Limonene, Limonenöl, alpha-Hexylzimtaldehyd, 1,3,4,6,7,8-Hexahydro-4,6,6,7,8,8-hexamethylcyclopentagamma-2-benzopyran, Terpinylacetat, Ethylenbrassylat. 1,2,3,4,5,6,7,8-octahydro-2,3,8,8-tetramethyl-2-Acetonaphthon, Amylsaliclyat, Terpineol, Cedrol, Vanillin. Methyldihdrojasmonat, Sclareoat, 2-lsobutyl-4-hydroxy-4-methyltetrahydropyran, 3-Methyl-5-phenyl-1-pentanol, Hexylsalicylat, Tetrahydrolinalool, Ethyllinalool, p-t-Butyl-alpha-methyldihydrozimtaldehyd, Benzylacetat, Orangenöl, 7-Acetyl-1,1,3,4,4,6-hexamethyltetralin, 2-tert-Pentyl-cyclohexylacetat, Lavandrinöl, Citronellol, Anisalkohol, Linaool, Heliotropin, d-Limonen, Benzylbenzoat, Amylcinnamylalkohol, Benzylcinnamat, Geraniol, Citral, Eugenol, Isoeugenol Famesol, Methyloctinoat, Linalylacetat, Methylcedrylketon, 4-t-Butylcyclohexylacetat, 3,7-Dimethyl-2,6-octadien-1-ol, Ethylenbrassylat, 3-Methyl-5-phenyl-1-pentanol, Terpinylacetat, Linalylacetat, 2-Isobutyl-4-hydroxy-4-methyltetra-hydrofuran, alpha-Amylzimtaldehyd.

Besonders vorteilhaft sind erfindungsgemäße Zubereitungen, dadurch gekennzeichnet, daß die sie einen oder mehrere Riechstoffe enthalten, gewählt aus der Gruppe Methylbenzoat ,3,7-Dimethyl-6-octen-1-ol, 2,6-Dimethyl-7-octen-2-ol, Hydraxycitronellal, omega-Pentadecalacton, Linalool, Coumarin, Bergamotteöl, Lavandinöl, Lavendelöl, Benzylsalicylat, Phenethylalkohol, Linalylacatat, Patchouliöl, alpha-Isomethylionon, Orangenterpene, 4-(4-hydroxy-4-methylpentyl)-3-cyclohexene-1-carboxyaldehyd, Methylcedrylketon, d-Limonene, Limonenöl, alpha-Hexylzimtaldehyd, 1,3,4,6,7,8-Hexahydro-4,6,6,7,8,8-hexamethylcyclopentagamma-2-benzopyran. Terpinylacetat, Ethylenebrassylat, 1,2,3,4,5,6,7,8-octahydro-2,3,8,8-tetramethyl-2-Acetonaphthon, Amylsaliclyat, Terpineol, Cedrol, Vanillin, Methyldihdrojasmonat, Sclareoat, 2-Isobutyl-4-hydroxy-4-methyltetrahydropyran, 3-Methyl-5-phenyl-1-pentanol, Hexylsalicylat, Tetrahydrolinalool, Ethyllinalool, p-t-Butyl-alpha-methyldihydrozimtaldehyd, Benzylacetat, Orangenöl, 7-Acetyl-1,1,3,4,4,6-hexamethyltetralin, 2-tert-Pentyl-cyclohexylacetat, Lavandrinöl, Citronellol, Anisalkohol, Linaool, Heliotropin, d-Limonen, Benzylbenzoat, Amylcinnamylalkohol, Benzylcinnamat, Geraniol, Citral, Eugenol, Isoeugenol Farnesol, Methyloctinoat, Linalylacetat, Methylcadrylketon, 4-t-Butylcyclohexylacetat, 3,7-Dimethyl-2,6-octadien-1-ol, Ethylenbrassylat, 3-Methyl-5-phenyl-1-pentanol, Terpinylacetat, Linalylacetat, 2-Isobutyl-4-hydroxy-4-methyltetrahydrofuran, alpha-Amylzimtaldehyd, und zwar in einer Gesamtmenge von 0,005 bis 0,5 Gewichts-%, bezogen auf das Gesamtgewicht der Zubereitung enthalten.

Gashaltige kosmetische Zubereitungen sind an sich bekannt und bereits in zahlreichen Patenten beschrieben worden. Als Gase werden beispielsweise Sauerstoff, fluorierte Gase, Kohlendioxid, Luft, Stickstoff und dergleichen verwendet.

Eine Möglichkeit zur Stabilisierung von Gasen in kosmetischen oder dermatologischen Zubereitungen besteht z. B. darin, eine O/W-Emulsion herzustellen, welche anschließend mit Gas beaufschlagt wird. Derartige Formulierungen sind auch als Mousse bekannt (beispielsweise WO 2002-474258-A1). Nachteilig an diesen Zubereitungen des Standes der Technik ist, dass das Gas nur durch den Einsatz von Emulgatoren stabilisiert werden kann. Ferner nachteilig ist, dass das Gas bei der topischen Applikation oder bereits bei Lagerung bei 40 °C oder mehr (beispielsweise im Auto oder am Strand) in die Atmosphäre entweichen kann. Ferner ist die Beladungskapazität derartiger Zubereitungen meistens eher gering, so dass sich physiologische Effekte nach der Applikation nicht einstellen.

Aus dem Stand der Technik sind kosmetische Zubereitungen, die Sauerstoff enthalten, an sich bekannt.

Die WO 02/05754 beschreibt extern applizierbare Zubereitungen, die einen Sauerstoffträger enthalten, der in einer lipoiden Emulsion molekulardispers eingearbeitet ist, sowie deren Verwendung zur externen Behandlung 1 Prävention von Sauerstoffrnangelzuständen der Haut.

Die WO 02/05754 offenbart O/W-Emulsionen, die als einen Saverstofftransport durch die Lipidschicht der Haut adäquat gewährleisten sollen.

In der US 5851544 wird beschrieben, dass Hautpflegezubereitungen, welche das Hautsauerstoffniveau erhöhen, wünschenswert seien, da der Sauerstoffgehalt in der Haut niedriger sei als in anderen Körperregionen und darüber hinaus mit zunehmendem Alter abnehme. Aufgabe der US 5851544 ist es daher, den Sauerstoffgehalt der Haut langfristig zu steigern bzw. Sauerstoffmangelzustände in der Haut zu beheben.

In der DE 10333710 werden kosmetische, dermatologische oder pharmazeutische Zubereitungen auf Basis von Lipiden und/oder Lipid/Wachs-Gemischen, welche ein Gas bzw. ein Gasgemisch enthalten, beschrieben.

Die DE 10342449, DE 102005011498, DE 102005011457 und DE 102206011459 offenbaren kosmetische Zubereitungen enthaltend molekularen Sauerstoff. Auch hierin werden bevorzugt O/W-Emulsionen beschrieben. Die Herstellung der sauerstoffhaltigen O/W-Emulsionen erfolgt indem eine Vordispergierung des anorganischen Gelbildners und Quellung des Hydrokolloides unter Rühren in der Wasserphase stattfindet. Eine Vereinigung der auf 75 °C aufgeheizten Fettphase mit der auf 70 °C aufgeheizten Wasserphase und Zugabe der partikulären hydrophoben, hydrophobisierten Festkörpersubstanzen unter Rühren. Es erfolgt dann Homogenisierung bei 65 °C und 45 min Rühren unter Vakuum und Kühlung. Die Zugabe der Additive erfolgt bei 30 °C, die Homogenisierung bei 27 °C. Anschließend wird die Begasung der Emulsion bei 7-8 bar mit Sauerstoff durchgeführt.

Der Stand der Technik konnt allerdings die vorteilhaften Eigenschaften des Suaerstoffes auf die Verträglichkeit von Zubereitungen, die sich durch einen Gehalt an Parfümbestandteiten auszeichnen, nicht vorwegahnen lassen.

Die Herstellung sauerstoffhaltiger kosmetischer Zubereitungen und insbesondere der Schritt der Sauerstoffzufuhr erfolgt in allen aufgeführten Druckschriften des Standes der Technik dabei unter Kühlung, bei Raumtemperatur bzw. bei maximal 30°C.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindugsgemäß vorteilhaft, Folsäure und/oder deren Derivate in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Die erfindungsgemäßen kosmetischen und dermatologischen Zubereitungen können kosmetische Hiffsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol. Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodisparsionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältem versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

### Beispielrezeptur 1:

| *Inhaltsstoff* | *Einsatzkonzentration in* % |
|---|---|
| Sucrose Polystearat + hydrogeniertes Polyisobuten | 1,5 |
| Kaliumcetylphosphat + hydrogenierte Palmglyceride | 0,8 |
| Glycerylstearat | 2 |
| Vaseline | 2 |
| Isopropyrlstearat | 2 |
| Macadamiaöl | 2 |
| Octyldodecanol | 1 |
| Ethylhexylsalicylat | 1 |
| Homosalat | 1,5 |
| Titandioxid | 2 |
| EDTA | 1,2 |
| Butylmethoxydibenzoylmethan | 1 |
| DHA | 2 |
| Vitamin E Acetat | 1 |
| Natronlauge | q.s. (pH-Wert auf ca.5) |
| Tapiocastärke | 1,5 |
| Glycerin | 12,5 |
| Butylenglycol | 4 |
| Iodopropinylbutylcarbamat | 0,3 |
| Parfum | 0,5 |

Dabei besteht das Parfumöl aus folgenden Bestandteilen:
18% 3,7-Dimethyl-6-octen-1-ol, 15% 2,6-Dimethyl-7-octen-2-ol, 15% Hydroxycitronellal, 10% omega-Pentadecalactone, 9% Linalool, 8% Coumarin, 7% Bergamotteöl, 7% alpha-Amylcinnamaldehyd, 6% Lavandinöl, 5% Lavendelöl

Nach Herstellung wird die auf ca. pH 5 eingestellte Zubereitung mit 18% Sauerstoff begast.

### Beispielrezeptur 2:

| *Inhaltsstoff* | *Einsatzkonzentration in* % |
|---|---|
| Glycerylstearatsitrat | 4 |
| Myristylmyristat | 2 |
| Caprylsäure/Caprinsäuretriglyceride | 3 |
| Traubenkernöl | 0,5 |
| Jojobaöl | 0,5 |
| Butylenglycoldicaprylat/dicaprat | 2 |
| Shea Butter | 3 |
| Dimethicon | 4 |
| Dimethicon/Vinyl Dimethicon Crosspolymer | 1 |
| Cera alba | 1 |
| Acrylate/C10-30 Alkylacrylatcrosspolymer | 0,5 |
| Nylon-12 | 1 |
| Q10 | 0,4 |
| 1,2-Pentandiol | 1 |
| Glycerin | 8 |
| Phenoxyethanol | 0,5 |
| Methylparaben | 0,1 |
| Ethylparaben | 0,1 |
| Parfum | 0,2 |

Dabei besteht das Parfumöl aus folgenden Bestandteilen:
20% Benzylsalicylat, 10% Phenethylalkohol, 10% Linalylacatat, 10% Patchouliöl, 10% alpha-Isomethylionon, 10% Orangenterpene, 9% Lavendelöl, 8% 4-(4-hydroxy-4-methylpentyl)-3-cyclohexene-1-carboxyaldehyd, 7% Methylcedrylketon, 3% d-Limonene, 2% Limonenöl, 1% Coumarin

Nach Herstellung wird die O/W-Emulsion mit 22% Sauerstoff begast.

### Beispielrezeptur 3:

| *Inhaltsstoff* | *Einsatzkonzentration in %* |
|---|---|
| Natriumstearoylglutamat | 2,5 |
| Cetylalkohol | 2 |
| Cetylstearylalkohol | 2 |
| Nachtkerzenöl | 1 |
| Dimethicon | 2 |
| Cyclomethicon | 3 |
| Dimethiconol | 1 |
| Isopropylstearat | 1 |
| Dicaprylyl Ether | 3 |
| Ethylhexylmthoxycinnamat | 5 |
| Ethanol | 3 |
| Diazolidinylhamstoff | 0,3 |
| Glycerin | 15 |
| BHT | 0,6 |
| 1,2 Octandiol | 0.4 |
| Folsäure | 0,4 |
| Kreatin | 0,4 |
| Parfum | 0,6 |

Dabei besteht das Parfumöl aus folgenden Bestandteilen:
15% alpha-Hexylcinnamaldehyd, 15% 1,3,4.6,7,8-Hexahydro-4,6,6,7,8,8-hexamethylcyclopentagamma-2-benzopyran, 12% Terpinylacetat, 10% Ethylenebrassylat, 11% 1,2,3,4,5,6,7,8-octahydro-2,3,8,8-tetramethyl-2-Acetonapthon, 8% Amylsaliclyat, 8% Terpineol, 7% Methyl-alpha-ionon, 4% Coumarin, 4% Lavendelöl, 3% Cedrol, 3% Vanillin

Nach Herstellung wird die O/W-Emulsion mit 11,5% Sauerstoff begast.

### Beispielrezeptur 4:

| *Inhaltsstoff* | *Einsatzkonzentration in* % |
|---|---|
| Glycerylisostearat | 3 |
| Myristylmyristat | 2 |
| Octyldodecanol | 2 |
| Babassuöl | 1 |
| Avocadoöl | 1 |
| Hydrierte Cocoglyceride | 3 |
| Cyclomethicon | 6 |
| Butylmethoxydibenzoylmethan | 0,7 |
| Phenylbenzimidazolsulionsäure | 2 |
| Farbpigmente wie Eisenoxide | 2 |
| Ammoniumacryloyldimethytaurat/VP Copolymer | 0,9 |
| Sojabohnen-Extrakt | 1 |
| Dioic Acid | 0,5 |
| Vitamin E Acetat | 0,7 |
| 1,2-Hexandiol | 0,3 |
| Glycerin | 5 |
| Ethylhexylglycerin | 4 |
| DMDM-Hyantoin | 0,6 |
| Natronlauge | q.s. |
| Parfum | 0,4 |

Dabei besteht das Parfumöl aus folgenden Bestandteilen;
20% Methyldihdrojasmonat, 18% Sclareoat, 16% 2-Isobutyl-4-hydroxy-4-methyltetrahydropyran, 16% 3-Methyl-5-phenyl-1-pentanol, 10% Hexylsalicylat, 7% Tetrahydrolinalool, 4% Ethyllinalool, 4% Limonenöl, 1% Vanillin

Nach Herstellung wird die O/W-Emulsion mit 26% Sauerstoff begast.

### Beispielrezeptur 5:

| *Inhaltsstoff* | *Einsatzkonzentration in %* |
|---|---|
| Stearinsäure | 4 |
| Myristylmyristat | 2 |
| C12-15 Alkylbenzoat | 3 |
| Paraffinöl | 1 |
| Cetyldimethicon | 4 |
| Isopropylpalmitat | 2 |
| Cyclomethicon | 1 |
| Talkum | 2 |
| Titandioxid | 4 |
| BHT | 0,8 |
| EDTA | 1,2 |
| Natronlauge | q.s. |
| Octocrylen | 2 |
| Chondrus Crispus | 0,4 |
| Carbomer | 0,2 |
| Panthenol | 2 |
| Calciumpantothenat | 0,5 |
| Glycerin | 7 |
| Methylpropandiol | 4 |
| 1,2-Hexandiol | 0.2 |
| Phenoxyethanol | 0.4 |
| Methylparaben | 0,1 |
| Ethylparaben | 0,1 |
| Propylparaben | 0,1 |
| Parfum | 0,5 |

Dabei besteht das Parfumöl aus folgenden Bestandteilen:
15% p-t-Butyl-alpha-methyldihydrozimtaldehyd, 15% Benzyl acetate, 13% Methyl dihydrojasmonate, 12% Orangenöl, 7% 7-Acetyl-1,1,3,4,4,6-hexamethyltetralin, 6% 2-tert-Pentyl-cyclohexyl acetate, 6% Benzylcinnamat, 5% Lavendelöl, 5% Lavandrin öl, 4% Bergamotteöl, 4% Farnesol, 3% 2-Isobutyl-4-hydroxy-4-methyltetrahydropyran, 3% Citronellol, 2% Anisalkohol

Nach Herstellung wird die O/W-Emulsion mit 28% Sauerstoff begast.

### Beispielrezeptur 6:

| *Inhaltsstoff* | *Einsatzkonzentration in %* |
|---|---|
| Glycerylstearatsitrat | 2 |
| Cetylalkohol | 2 |
| Cetytstaerylalkohol | 2 |
| Hydrogenierte Coco-Glyceride | 2 |
| C12-15 Alkylbenzoate | 2 |
| Cyclomethicon | 2 |
| Mandelöl | 2 |
| Octocrylen | 3 |
| Butylmethoxydibenzoylmethan | 0,5 |
| Carbomer | 0,8 |
| Süßholzwurzelextrakt | 0,2 |
| Vitamin E Acetat | 0,5 |
| BHT | 0,1 |
| EDTA | 1 |
| Methylpropandiol | 2 |
| Glycerin | 10 |
| Phenoxyethanol | 0,3 |
| Methylparaben | 0,15 |
| Ethylparaben | 0,15 |
| Propylparaben | 0,15 |
| Parfum | 0,4 |

Dabei besteht das Parfumöl aus folgenden Bestandteilen:
20% Hexylsalicylat, 10% Linaool, 10% Methyldihydrojasmonate, 10% Heliotropin, 10% Orange Terpene, 10% d-Limonene, 8% Cedrol, 5% Orangenöl, 5% Limonenöl, 5% Benzylbenzoate, 5% Vanillin, 2% Bergamotteöl,

Nach Herstellung wird die O/W-Emulsion mit 16% Sauerstoff begast,

### Beispielrezeptur 7:

| *inhaltsstoff* | *Einsatzkonzentration in %* |
|---|---|
| PEG-40-Stearat | 2 |
| Stearinsäure | 1 |
| Cetylalkohol | 2 |
| Dicaprylylcarbonat | 3 |
| Ethylhexyl Stearat | 2 |
| Sqalene | 1 |
| Butylenglycol Dicaprylate/Dicaprate | 3 |
| Cyclomethicon | 4 |
| Butylmethaxydibenzoylmethan | 4 |
| Ethylhexylmthoxycinnamat | 2 |
| EDTA | 1 |
| Vitamin E Acetat | 0,5 |
| Vitamin C | 1 |
| Natridmascorbylphosphate | 0,5 |
| Methylpropandiol | 5 |
| Glycerin | 15 |
| Polyaminopropyl Biguanide | 0,5 |
| Parfum | 0,7 |

Dabei besteht das Parfumöl aus folgenden Bestandteilen:
18% Hydroxycitronellal, 15% Methyl dihydrojasmonate, 15% Amylcinnamyl Alcohol, 14% Benzyl Cinnamate, 6% Citronellol, 6% Coumarin, 6% Geraniol, 5% Citral, 5% Eugenol, 5% Farnesol, 4% Methyl-Octynoate, 1% Linalool

Nach Herstellung wird die O/W-Emulsion mit 20% Sauerstoff begast.

### Beispielrezeptur 8:

| *Inhaltsstoff* | *Einsatzkonzentration in %* |
|---|---|
| Polyglyceryl-3 Methylglucose Distearat | 4 |
| Cetylalkohol | 2 |
| Glycerylstearat | 2 |
| Caprylsäure/Caprinsäuretriglyceride | 2 |
| Synthetisches Bienenwachs | 1 |
| Butylenglycol Dicaprylate/Dicaprate | 4 |
| Butylmethoxydibenzoylmethan | 2 |
| Benzophneone-3 | 2 |
| Octocrylene | 1 |
| Xanthan Gum | 1 |
| Vitamin E Acetat | 0,5 |
| Panthenol | 1 |
| EDTA | 1 |
| Glucosylrutin + Isoquercitrin | 0,5 |
| Glycerin | 7 |
| Phenoxyethanol | 0,3 |
| Methylparaben | 0,15 |
| Ethylparaben | 0,15 |
| Propylparaben | 0,15 |
| Parfum | 0,4 |

Dabei besteht das Parfumöl aus folgenden Bestandteilen:
10% Linalool, 10% Linatyl acetate, 10% Patchouliöl. 10% Hexyl salicylate, 10% Methyl *cedryl* ketone, 10% 4-t-Butylcyclohexylacetat, 10% 3,7-Dimethyl-2,6-octadien-1-ol, 10% Ethylenebrassylat, 5% 3-Methyl-5-phenyl-1-pentanol, 5% 2-Isobutyl-4-hydroxy-4-methyltetrahydronpyran, 5% Limonene, 5% Amyl salicylat

Nach Herstellung wird die O/W-Emulsion mit 12,4% Sauerstoff begast.

### Beispielrezeptur 9:

| *inhaltsstoff* | *Einsatzkonzentration in* % |
|---|---|
| Ceteareth-20 | 2 |
| Ceteareth-12 | 2 |
| Myristylmyristat | 3 |
| Stearylalkohol | 2 |
| Hydrogeniertes Lecithin | 1 |
| Tridecyl Stearat + Tridecyl Trimelliate | |
| + Dipentaerythrityl Hexacaprylate/Hexacaprat | 2 |
| Dicaprylylcarbonat | 2 |
| Hydrogeniertes Pflanzenöl | 2 |
| Cyclomethicon | 5 |
| Dimethicon | 5 |
| Q10 | 0,2 |
| Vitamin E Acetat | 0,5 |
| BHT | 0,5 |
| Natriumascorbylphosphat | 0,2 |
| Phenoxyethanol + Methylparaben + Ethylparaben + | |
| Butylparaben + Isobutylparaben + Propylparaben | 0,8 |
| Parfum | 1 |

Dabei besteht das Parfumöl aus folgenden Bestandteilen:
10% Linalool, 10% Orangenterpene, 10% Heliotropin, 10% Methyldihydrojasmonat, 10% Methyl cedrylketone, 10% Terpinylacetat, 10% Methyl-alpha-ionon, 10% omega-Pentadecatacton, 5% Lavadinöl, 5% Limonenöl, 5% Bergamotteöl, 5% 2-Isobutyl-4-hydroxy-4-methyltetrahydropyran

Nach Herstellung wird die O/W-Emulsion mit 28% Sauerstoff begast.

### Beispielrezeptur 10:

| *Inhaltsstoff* | *Einsatzkonzentration in %* |
|---|---|
| Glycerylstearat SE | 2 |
| Cetylstearylalkohol | 3 |
| Myristylalkohol | 1 |
| Caprylsäure/Caprinsäuretriglyceride | 3 |
| Shea butter | 2 |
| Dicaprylylcarbonat | 2 |
| Butylenglycol Dicaprylate/Dicaprate | 4 |
| Butylmethoxydibenzoylmethan | 3 |
| Ethylhexylmethaxycinnamat | 3 |
| Octocrylen | 3 |
| Cetyldimethicon | 4 |
| Vitamin E Acetat | 1 |
| BHT | 0,5 |
| Methylpropandiol | 4 |
| Ethythexylglycerin | 4 |
| 1,2-Pentandiol | 0,3 |
| 1,2-Hexandiol | 0,3 |
| 1,2-Octandiol | 0,3 |
| Parfum | 0,2 |

Dabei besteht das Parfumöl aus folgenden Bestandteilen:
10% Phenethylalkohol, 10% Linalylacetat, 10% alpha-Hexylzimtaldehyd, 10% Benzylacatat, 10% Ethylenbrassylat, 10% Patchouliöl, 10% Hydroxycitronellal, 5% Lavendelöl, 5% Terpineol, 5% 2-Isobutyl-4-hydroxy-4-methyltetrahydrofuran, 5% d-Limonen, 5% alpha-Amylcinnamaldehyd, 5% Vanillin

Nach Herstellung wird die O/W-Emulsion mit 8% Sauerstoff begast.

## Patentansprüche

1. Kosmetische Zubereitungen mit einem Gehalt an molekularem Sauerstoff und einem oder mehreren Parfümstoffen, insbesondere gewählt aus der Gruppe der Verbindungen Methylbenzoat ,3,7 -Dimethyl-6-octen-1-ol, 2,6-Dimethyl-7-octen-2-ol, Hydroxycitronellal, omega-Pentadecalacton, Linalool, Coumarin, Bergamotteöl, Lavandinöl, Lavendelöl, Benzylsalicylat, Phenethylalkohol, Linalylacatat, Patchouliöl, alpha-Isomethylionon, Orangenterpene, 4-(4-hydroxy-4-methylpentyl)-3-cyclohexene-1-carboxyaldehyd, Methylcedrylketon, d-Limonene, Limonenöl, alpha-Hexylzimtaldehyd, 1.3,4.6,7,8-Hexahydro-4,6,6,7,8,8-hexamethylcyclopentagemma-2-benzopyran, Terpinylacetat, Ethylenbrassylat, 1,2,3,4,5,6,7,8-octahydro-2,3,8,8-tetramethyl-2-Acetonaphthon, Amylsaliclyat, Terpineol, Cedrol, Vanillin, Methytdthdrojasmonat, Sclareoat, 2-Isabutyl-4-hydroxy-4-methyltetrahydropyran, 3-Methyl-5-phenyl-1-pentanol, Hexylsalicylat, Tetrahydrolinalool, Ethyllinalool, p-t-Butyl-alpha-methyldihydrozimtaldehyd, Benzylacetat, Orangenöl. 7-Acetyl-1,1,3,4,4,6-hexamethyltetralin, 2-tert-Pentyl-cyclohexylacetat, Lavandrinöl, Citronellol, Anisalkohol, Linaool, Heliotropin, d-Limonen, Benzylbenzoat, Amylcinnamylalkohol, Benzylcinnamat, Geraniol, Citral, Eugenol. Isoeugenol Farnesol, Methyloctinoat, Linalylacetat, Methylcedrylketon, 4-t-Butylcyclohexytacetat, 3,7-Dimethyl-2,6-octadien-1-ol, Ethylenbrassylat, 3-Methyl-5-phenyl-1-pentanol, Terpinylacetat, Linalylacetat, 2-lsobutyl-4-hydroxy-4-methyltetra-hydrofuran, alpha-Amylzimtaldehyd

2. Zubereitungen nach Anspruch 1, **dadurch gekennzeichnet, daß** die sie einen oder mehrere Riechstoffe enthalten, gewählt aus der Gruppe Methylbenzoat ,3,7-Dimethyl-6-octen-1-ol, 2,6-Dimethyl-7-octen-2-ol, Hydroxycitronellal, omega-Pentadecalacton, Linalool, Coumarin, Bergamotteöl, Lavandinöl, Lavendelöl, Benzylsalicylat, Phenethylalkohol, Linalylacatat, Patchouliöl, alpha-Isomethylionon, Orangenterpene, 4-(4-hydroxy-4-methylpentyl)-3-cyclohexene-1-carboxyaldehyd, Methylcedrylketon, d-Limonene, Limonenöl, alpha-Hexylzimtaldehyd, 1,3,4,6,7,8-Hexahydro-4,6,6,7,8,8-hexamathylcyclopentagamma-2-benzopyran, Terpinylacetat, Ethylenebrassylat, 1,2,3,4,5,6,7,8-octahydro-2,3,8,8-tetramethyl-2-Acetonaphthon, Amylsaliclyat, Terpineol, Cedrol, Vanillin, Methyldihdrojasmonat, Sclareoat, 2-Isobutyl-4-hydroxy-4-methyltetrahydropyran, 3-Methyl-5-phenyl-1-pentanol, Hexylsalicylat, Tetrahydrolinalool, Ethyllinalool, p-t-Butyl-alpha-methyldihydrozimtaldehyd, Benzylacetat, Orangenöl, 7-Acetyl-1,1,3,4,4,6-hexamethyltetralin, 2-tert-Pentyl-cyclohexylaretat, Lavandrinöl, Citronellol, Anisalkohol, Linaool, Heliotropin, d-Limonen, Benzylbenzoat, Amylcinnamylalkohol, Benzylcinnamat, Geraniol, Citral, Eugenol, Isoeugenol Farnesol, Methyloctinoat, Linalylacetat, Methylcedrylketon, 4-t-Butylcyclohexylacetat, 3,7-Dimethyl-2,6-octadien-1-ol, Ethylenbrassylat, 3-Methyl-5-phenyl-1-pentanol, Terpinylacetat, Linalylacetat, 2-Isobutyl-4-hydroxy-4-methyltetrahydrofuran, alpha-Amylzimtaldehyd, und zwar in einer Gesamtmenge von 0,005 bis 0,5 Gewichts-%, bezogen auf das Gesamtgewicht der Zubereitung enthalten.

3. Zubereitung nach einem der vorstehenden Ansprüche, enthaltend 5-30 Volumen-% Sauerstoff, vorzugsweise 10-20 Volumen-%, insbesondere bevorzugt 12-17 Volumen-.%
